# EUROPEAN PATENT SPECIFICATION

(11) **EP 1 001 783 B1**
(45) Date of publication and mention of the grant of the patent: **05.10.2005**
(21) Application number: 98935127.5
(22) Date of filing: 16.07.1998
(51) Int. Cl.: A61K 31/64, A61K 31/44, A61K 31/155

(54) **TREATMENT OF DIABETES WITH THIAZOLIDINEDIONE, INSULIN SECRETAGOGUE AND DIGUANIDE**
BEHANDLUNG DER DIABETES MIT THIAZOLIDINDIONE, INSULIN SEKRETIONFÖRDERER UND BIGUANIDE
TRAITEMENT DU DIABETE AVEC DU THIAZOLIDINEDIONE, UN SECRETAGOGUE D'INSULINE ET DU BIGUANIDE

(30) Priority: 18.07.1997 GB 9715295
(43) Date of publication of application: 24.05.2000
(73) Proprietor: SMITHKLINE BEECHAM PLC, Middlesex TW8 9GS (GB)
(72) Inventor: BUCKINGHAM, Robin Edwin, SmithKline Beecham Pharm., Harlow, Essex CM19 5AW (GB); SMITH, Stephen Alistair, SmithKline Beecham Pharm., Harlow, Essex CM19 5AW (GB)
(74) Representative: Rutter, Keith
(86) International application number: PCT/GB1998/002110
(87) International publication number: WO 1999/003477

(56) References cited:
- EP-A- 0 749 751
- WO-A-93/03724
- WO-A-96/09823
- KAPPEL C. ET AL: "Type 2 diabetes: Update on therapy" COMPREHENSIVE THERAPY, vol. 24, no. 6-7, June 1998, pages 319-326, XP002080974
- ELSON DF ET AL: "Therapy for type 2 diabetes mellitus [see comments]" WMJ, MAR 1998, 97 (3) P49-54, XP002081122 UNITED STATES
- SCHEEN AJ ET AL: "Oral antidiabetic agents. A guide to selection." DRUGS, FEB 1998, 55 (2) P225-36, XP002080821 NEW ZEALAND
- WOLFFENBUTTEL B. ET AL: "New treatments for patients with type 2 diabetes mellitus" POSTGRAD MED, vol. 72, pages 657-662, XP002081123

## Description

This invention relates to a method of treatment, in particular to a method for the treatment of diabetes mellitus, especially non-insulin dependent diabetes (NIDDM) (or Type 2 diabetes) and conditions associated with diabetes mellitus.

Insulin secretagogues are compounds which promote increased secretion of insulin by the pancreatic beta cells.

The sulphonylureas are well known examples of insulin secretagogues. The sulphonylureas act as antihyperglycaemic agents and are used in the treatment of Type 2 diabetes). Examples of sulphonylureas include glibenclamide, glipizide, gliclazide, glimepiride, tolazamide and tolbutamide.

Biguanide antihyperglycaemic agents are commonly used in the treatment of Type 2 diabetes). 1,1 - Dimethylbiguanidine (or Metformin) is an example of a biguanide antihyperglycaemic agent.

European Patent Application. Publication Number 0,306,228 relates to certain' thiazolidinedione derivatives disclosed as having antihyperglycaemic and antihyperlipidaemic activity. One particular thiazolidinedione disclosed in EP 0306228 is 5-[4-[2-(N-methyl-N-(2-pyridyl)amino)ethoxy]benzyl]thiazolidine-2,4-dione (hereinafter 'Compound (I)'). WO94/05659 discloses certain salts of Compound (I) including the maleate salt.

Compound (I) is an example of a class of anti-hyperglycaemic agents known as 'insulin sensitisers'. In particular Compound (I) is a thiazolidinedione insulin sensitiser.

European Patent Applications. Publication Numbers: 0008203, 0139421, 0032128, 0428312, 0489663, 0155845, 0257781, 0208420, 0177353, 0319189, 0332331, 0332332, 0528734, 0508740; International Patent Application, Publication Numbers 92/18501, 93/02079, 93/22445 and United States Patent Numbers 5104888 and 5478852, also disclose certain thiazolidinedione insulin sensitisers.

Another series of compounds generally recognised as having insulin sensitiser activity are those typified by the compounds disclosed in International Patent Applications, Publication Numbers WO93/21166 and WO94/01420. These compounds are herein referred to as 'acyclic insulin sensitisers'. Other examples of acyclic insulin sensitisers are those disclosed in United States Patent Number 5232945 and International Patent Applications, Publication Numbers WO92/03425 and WO91/19702.

Examples of other insulin sensitisers are those disclosed in European Patent Application, Publication Number 0533933, Japanese Patent Application Publication Number 05271204 and United States Patent Number 5264451.

It is now surprisingly indicated that Compound (I) in combination with an insulin secretagogue and a biguanide antihyperglycaemic agent provides a particularly beneficial effect on glycaemic control, such combination is therefore particularly useful for the treatment of diabetes mellitus, especially Type 2 diabetes and conditions associated with diabetes mellitus. The treatment is also indicated to proceed with minimum side effects.

A pharmaceutical composition for the treatment of diabetes mellitus and conditions associated with diabetes mellitus in a mammal with a beneficial effect on glycaemic control, which comprises 5-[4-[2-(N-methyl-N-(2-pyridyl) amino)ethoxy]benxyl]thiazolidine-2,4-dione (Compound I) or pharmaceutically acceptable form thereof in an amount of 2 to 12 mg, an insulin secretagogue and a biguanide antihyperglycaemic agent.

The pharmaceutical formulation of the invention is useful in a method for the treatment of diabetes mellitus, especially Type 2 diabetes, and conditions associated with diabetes mellitus, in a mammal such as a human, which method comprises administering an effective non-toxic and pharmaceutically acceptable amount of an insulin sensitiser, an insulin secretagogue and a biguanide antihyperglycaemic agent, to a mammal in need thereof.

The method comprises either co-administration of the insulin sensitiser, an insulin secretagogue and a biguanide antihyperglycaemic agent or sequential administration thereof.

Co-administration includes administration of a formulation which includes an insulin sensitiser, an insulin secretagogue and a biguanide antihyperglycaemic agent or the essentially simultaneous administration of separate formulations of each agent.

In another aspect the invention provides the use of an insulin sensitiser, such as Compound (I), an insulin secretagogue and a biguanide antihyperglycaemic agent, in the manufacture of a composition for the treatment of diabetes mellitus, especially Type 2 diabetes and conditions associated with diabetes mellitus.

A suitable insulin sensitiser is a thiazolidinedione insulin sensitiser.

A suitable thiazolidinedione insulin sensitiser is Compound (I).

Other suitable thiazolidinedione insulin sensitisers include (+) -5-[[4-[(3,4-dihydro-6-hydroxy-2, 5, 7, 8-tetramethyl-2H-1-benzopyran-2-yl)methoxy]phenyl]methyl]-2,4-thiazolidinedione (or troglitazone), 5-[4-[(1-methylcyclohexyl)methoxy]benzyl] thiazolidine-2,4-dione (or ciglitazone), 5-[4-[2-(5-ethylpyridin-2-yl)ethoxy]benzyl] thiazolidine-2,4-dione (or pioglitazone) or 5-[(2-benzyl-2,3-dihydrobenzopyran)-5-ylmethyl)thiazolidine-2,4-dione (or englitazone)

Suitable insulin secretagogues include sulphonylureas.

A suitable biguanide antihyperglycaemic agent is metformin, buformin or phenformin, especially metformin.

The method comprises the administration of 2 to 12 mg of Compound (I), especially when administered per day.

Particularly, the method comprises the administration of 2 to 4, 4 to 8 or 8 to 12 mg of Compound (I) per day.

Particularly, the method comprises the administration of 2 to 4mg of Compound (I), especially when administered per day.

Particularly, the method comprises the administration of 4 to 8mg of Compound (I), especially when administered per day.

Particularly, the method comprises the administration of 8 to 12 mg of Compound (I), especially when administered per day.

Preferably, the method comprises the administration of 2 mg of Compound (I), especially when administered per day.

Preferably, the method comprises the administration of 4 mg of Compound (I), especially when administered per day.

Preferably, the method comprises the administration of 8 mg of Compound (I), especially when administered per day.

It will be understood that the insulin sensitiser, such as compound (I), the insulin secretagogue and the biguanide antihyperglycaemic agent are each administered in a pharmaceutically acceptable form, including pharmaceutically acceptable derivatives such as pharmaceutically acceptable salts, esters and solvates thereof, as appropriate. In certain instances herein the names used for the relevant insulin secretagogues and the biguanide antihyperglycaemic agents may relate to a particular pharmaceutical form of the relevant active agent: It will be understood that all pharmaceutically acceptable forms of the active agents per se are encompassed by this invention.

Suitable pharmaceutically acceptable salted forms of the insulin sensitisers, such as Compound (I), include those described in the above mentioned patents and patent applications such as in EP 0306228 and WO94/05659 for Compound (I). A preferred pharmaceutically acceptable salt for Compound (I) is a maleate.

Suitable pharmaceutically acceptable solvated forms of the insulin sensitisers, such as Compound (I), include those described in the above mentioned patents and patent applications, such as in EP 0306228 and WO94/05659 for Compound (I), in particular hydrates.

Suitable pharmaceutically acceptable forms of the insulin secretagogue and the biguanide antihyperglycaemic agent depend upon the particular compound used but include known pharmaceutically acceptable forms of the particular compound

Suitable sulphonylureas include glibenclamide, glipizide, gliclazide, glimepiride, tolazamide and tolbutamide.

Further sulphonylureas include acetohexamide, carbutamide, chlorpropamide, glibomuride, gliquidone, glisentide, glisolamide, glisoxepide, glyclopyamide and glycylamide.

Further suitable insulin secretagogues include repaglinide. chosen. Such derivatives are found or are referred to in standard reference texts such as the British and US Pharmacopoeias, Remington's Pharmaceutical Sciences (Mack Publishing Co.), Martindale The Extra Pharmacopoeia (London, The Pharmaceutical Press) (for example see the 31 st Edition page 341 and pages cited therein).

The insulin sensitisers, such as Compound (I) or, a pharmaceutically acceptable salt thereof, or a pharmaceutically acceptable solvate thereof, may be prepared using known methods, for example those disclosed in the above mentioned patents and patent applications, such as EP 0306228 and WO94/05659 for Compound (I). The disclosures of the above mentioned patents and patent applications, such as EP 0306228 and WO94/05659, are incorporated herein by reference.

Compound (I) may exist in one of several tautomeric forms, all of which are encompassed by the term Compound (I) as individual tautomeric forms or as mixtures thereof. Compound (I) contains a chiral carbon atom, and hence can exist in up to two stereoisomeric forms, the term Compound (I) encompasses all of these isomeric forms whether as individual isomers or as mixtures of isomers, including racemates.

The insulin secretagogue and biguanide antihyperglycaemic agent of choice is prepared according to known methods, such methods are found or are referred to in standard reference texts, such as the British and US Pharmacopoeias, Remington's Pharmaceutical Sciences (Mack Publishing Co.), Martindale The Extra Pharmacopoeia (London, The Pharmaceutical Press) (for example see the 3 1 st Edition page 341 and pages cited therein).

When used herein the term 'conditions associated with diabetes' includes conditions associated with diabetes mellitus itself and complications associated with diabetes mellitus. Also included in 'conditions associated with diabetes' are those conditions associated with the pre-diabetic state.

When used herein the term 'conditions associated with the pre-diabetic state' includes conditions such as insulin resistance, including hereditary insulin resistance, impaired glucose tolerance and hyperinsulinaemia.

'Conditions associated with diabetes mellitus itself' include hyperglycaemia, insulin resistance, including acquired insulin resistance. Further conditions associated with diabetes mellitus itself include hypertension and cardiovascular disease, especially atherosclerosis and conditions associated with insulin resistance. Conditions associated with insulin resistance include polycystic ovarian syndrome and steroid induced insulin resistance and gestational diabetes.

'Complications associated with diabetes mellitus' includes renal disease, especially renal disease associated with Type 2 diabetes, neuropathy and retinopathy.

Renal diseases associated with Type 2 diabetes include nephropathy, glomerulonephritis, glomerular sclerosis, hypertensive nephrosclerosis and end stage renal disease. Additional renal diseases associated with Type 2 diabetes include nephrotic syndrome.

For the avoidance of doubt, when reference is made herein to scalar amounts, including mg amounts, of Compound (I) in a pharmaceutically acceptable form, the scalar amount referred to is made in respect of Compound (I) *per se*: For example 2 mg of Compound (I) in the form of the maleate salt is that amount of maleate salt which contains 2 mg of Compound (I).

Diabetes mellitus is preferably Type 2 diabetes.

The particularly beneficial effect on glycaemic control provided by the treatment of the invention is indicated to be a synergistic effect relative to the control expected for the sum of the effects of the individual active agents.

Suitably the insulin sensitiser is the agent of first administration. Glycaemic control may be characterised using conventional methods, for example by measurement of a typically used index of glycaemic control such as fasting plasma glucose or glycosylated haemoglobin (HbA1c). Such indices are determined using standard methodology, for example those described in: Tuescher A, Richterich, P., Schweiz. med. Wschr. 101 (1971), 345 and 390 and Frank P., 'Monitoring the Diabetic Patent with Glycosolated Hemoglobin Measurements', Clinical Products 1988

In a preferred aspect, the dosage level of each of the active agents when used in accordance with the treatment of the invention will be less than would have been required from a purely additive effect upon glycaemic control.

There is also an indication that the treatment of the invention will effect an improvement, relative to the individual agents, in the levels of advanced glycosylation end products (AGEs), leptin and serum lipids including total cholesterol, HDL-cholesterol, LDL-cholesterol including improvements in the ratios thereof, in particular an improvement in serum lipids including total cholesterol, HDL-cholesterol, LDL-cholesterol including improvements in the ratios thereof.

As used herein the term 'pharmaceutically acceptable' embraces both human and veterinary use: for example the term 'pharmaceutically acceptable' embraces a veterinarily acceptable compound.

In the method, the active medicaments are preferably administered in pharmaceutical composition form. As indicated above, such compositions can include all medicaments or one only of the medicaments.

Accordingly, in one aspect the present invention also provides a pharmaceutical composition comprising an insulin sensitiser, such as Compound (I) and especially 2 to 12 mg thereof, an insulin secretagogue and a biguanide antihyperglycaemic agent and a pharmaceutically acceptable carrier therefor.

Such compositions may be prepared by admixing an insulin sensitiser, such as Compound (I) and especially 2 to 12 mg thereof, an insulin secretagogue and a biguanide antihyperglycaemic agent and a pharmaceutically acceptable carrier therefor.

Usually the compositions are adapted for oral administration. However, they may be adapted for other modes of administration, for example parenteral administration, sublingual or transdermal administration.

The compositions may be in the form of tablets, capsules, powders, granules, lozenges, suppositories, reconstitutable powders, or liquid preparations, such as oral or sterile parenteral solutions or suspensions.

In order to obtain consistency of administration it is preferred that a composition of the invention is in the form of a unit dose.

Unit dose presentation forms for oral administration may be tablets and capsules and may contain conventional excipients such as binding agents, for example syrup, acacia, gelatin, sorbitol, tragacanth, or polyvinylpyrrolidone; fillers, for example lactose, sugar, maize-starch, calcium phosphate, sorbitol or glycine; tabletting lubricants, for example magnesium stearate; disintegrants, for example starch, polyvinylpyrrolidone, sodium starch glycollate or microcrystalline cellulose; or pharmaceutically acceptable wetting agents such as sodium lauryl sulphate.

The compositions are preferably in a unit dosage form in an amount appropriate for the relevant daily dosage.

Suitable dosages for the insulin sensitisers include those disclosed in the abovementioned patents and patent applications.

Suitable dosages, including unit dosages, of Compound (I) comprise 1, 2, 3, 4, 5, 6, 7, 8, 9, 10, 11 or 12 mg of Compound (I).

In the treatment the medicaments may be administered from 1 to 6 times a day, but most preferably 1 or 2 times per day.

Particular dosages of Compound (I) are 2mg/day, 4mg/day, including 2mg twice per day, and 8 mg/day, including 4mg twice per day.

Suitable dosages including unit dosages of the insulin secretagogue, such as the sulphonylurea, or the biguanide antihyperglycaemic agent, include the known dosages including unit doses for these compounds as described or referred to in reference text such as the British and US Pharmacopoeias, Remington's Pharmaceutical Sciences (Mack Publishing Co.), Martindale The Extra Pharmacopoeia (London, The Pharmaceutical Press) (for example see the 31 st Edition page 341 and pages cited therein).

Thus for the sulphonylureas, a typical daily dosage of glibenclamide is in the range of from 2.5 to 20 mg, for example 10mg twice per day or 20mg once per day; a typical daily dosage of glipizide is in the range of from 2.5 to 40 mg; a typical daily dosage of gliclazide is in the range of from 40 to 320 mg; a typical daily dosage of tolazamide is in the range of from 100 to 1000 mg; a typical daily dosage of tolbutamide is in the range of from 1000 to 3000 mg; a typical daily dosage of chlorpropamide is in the range of from 100 to 500 mg; and a typical daily dosage of gliquidone is in the range of from 15 to 180 mg.

Repaglinide may be taken in amounts, usually in the range of from 0.5mg to 4mg and usually with meals, up to a typical maximum daily dosage of 16mg per day.

With regard to the biguanide antihyperglycaemic agents, suitable dosages of metformin include up to 3000mg per day, in unit doses of 500mg (for example two or three times per day) or 850mg (for example two times per day), one example of a dosage for metformin is 500mg once building to five times per day.

The solid oral compositions may be prepared by conventional methods of blending, filling or tabletting. Repeated blending operations may be used to distribute the active agent throughout those compositions employing large quantities of fillers. Such operations are of course conventional in the art. The tablets may be coated according to methods well known in normal pharmaceutical practice, in particular with an enteric coating.

Oral liquid preparations may be in the form of, for example, emulsions, syrups, or elixirs, or may be presented as a dry product for reconstitution with water or other suitable vehicle before use. Such liquid preparations may contain conventional additives such as suspending agents, for example sorbitol, syrup, methyl cellulose, gelatin, hydroxyethylcellulose, carboxymethylcellulose, aluminium stearate gel, hydrogenated edible fats; emulsifying agents, for example lecithin, sorbitan monooleate, or acacia; non-aqueous vehicles (which may include edible oils), for example almond oil, fractionated coconut oil, oily esters such as esters of glycerine, propylene glycol, or ethyl alcohol; preservatives, for example methyl or propyl p-hydroxybenzoate or sorbic acid; and if desired conventional flavouring or colouring agents.

For parenteral administration, fluid unit dosage forms are prepared utilizing the compound and a sterile vehicle, and depending on the concentration used, can be either suspended or dissolved in the vehicle. In preparing solutions the compound can be dissolved in water for injection and filter sterilized before filling into a suitable vial or ampoule and sealing. Advantageously, adjuvants such as a local anaesthetic, a preservative and buffering agents can be dissolved in the vehicle. To enhance the stability, the composition can be frozen after filling into the vial and the water removed under vacuum. Parenteral suspensions are prepared in substantially the same manner, except that the Compound (I) is suspended in the vehicle instead of being dissolved, and sterilization cannot be accomplished by filtration. The compound can be sterilized by exposure to ethylene oxide before suspending in the sterile vehicle. Advantageously, a surfactant or wetting agent is included in the composition to facilitate uniform distribution of the compound.

Compositions may contain from 0.1% to 99% by weight, preferably from 10-60% by weight, of the active material, depending upon the method of administration.

Composition may, if desired, be in the form of a pack accompanied by written or printed instructions for use.

The compositions are prepared and formulated according to conventional methods, such as those disclosed in standard reference texts, for example the British and US Pharmacopoeias, 's Pharmaceutical Sciences (Mack Publishing Co.), Martindale The Extra Pharmacopoeia (London, The Pharmaceutical Press) and Harry's Cosmeticology (Leonard Hill Books) (for example see the 31st Edition page 341 and pages cited therein).

The present invention also provides a pharmaceutical composition comprising an insulin sensitiser, such as Compound (I) and especially 2 to 12 mg thereof, an insulin secretagogue and a biguanide antihyperglycaemic agent and a pharmaceutically acceptable carrier therefor, for use as an active therapeutic substance.

The invention also provides the use of Compound (I) in an amount of 2 to 12 mg , an insulin secretagogue and a biguanide antihyperglycaemic agent for the manufacture of a medicament for the treatment of diabetes mellitus and conditions associated with diabetes in a mammal..

A range of 2 to 4mg includes a range of 2.1 to 4, 2.2 to 4, 2.3 to 4, 2.4 to 4, 2.5 to 4, 2.6 to 4, 2.7 to 4, 2.8 to 4, 2.9 to 4 or 3 to 4mg.

A range of 4 to 8mg includes a range of 4.1 to 8, 4.2 to 8, 4.3 to 8, 4.4 to 8, 4.5 to 8, 4.6 to 8, 4.7 to 8, 4.8 to 8, 4.9 to 8, 5 to 8, 6 to 8 or 7 to 8mg.

A range of 8 to 12 mg includes a range of 8.1 to 12, 8.2 to 12, 8.3 to 12, 8.4 to 12, 8.5 to 12, 8.6 to 12, 8.7 to 12, 8.8 to 12, 8.9 to 12, 9 to 12, 10 to 12 or 11 to 12mg.

No adverse toxicological effects are expected for the compositions or methods of the invention in the abovementioned dosage ranges.

### Composition for compound (I)

| **Preparation of Concentrate:** Tabletting concentrate was prepared using the following materials | |
|---|---|
| **Ingredient** | **Quantity (%)** |
| Milled Compound (I) as maleate salt | 13.25 (pure maleate salt) |
| Sodium Starch Glycollate | 5.00 |
| Hydoxypropyl Methylcellulose 2910 | 5.00 |
| Microcrystalline Cellulose (Avicel PH102) | 20.0 |
| Lactose Monohydrate, regular grade | to 100 |
| Purified water | * |

| | |
|---|---|
| * Removed during processing. | |

The concentrate was then formulated into tablets using the following:

| | **Quantity (mg per Tablet)** | | | |
|---|---|---|---|---|
| **Tablet Strength** | **1.0mg** | **2.0mg** | **4.0mg** | **8.0mg** |
| **Active Ingredient:** | | | | |
| Compound (I) maleate Concentrate granules | 10.00 | 20.00 | 40.00 | 80.00 |

| **Other Ingredients:** | | | | |
|---|---|---|---|---|
| Sodium Starch Glycollate | 6.96 | 6.46 | 5.46 | 10.92 |
| Microcrystalline Cellulose (Avicel PH102) | 27.85 | 25.85 | 21.85 | 43.70 |
| Lactose monohydrate, (Pharmatose DCL15), | 104.44 | 96.94 | 81.94 | 163.88 |
| Magnesium Stearate | 0.75 | 0.75 | 0.75 | 1.50 |
| **Total Weight of Tablet Core** | 150.0 | 150.0 | 150.0 | 300.0 |
| Opadry | 4.5 | 4.5 | 4.5 | 9.0 |
| **Total Weight of Film Coated Tablet** | 154.5 | 154.5 | 154.5 | 309.0 |

Compositions for other active agents are as described in the above mentioned publications.

## Claims

1. A pharmaceutical composition for the treatment of diabetes mellitus and conditions associated with diabetes mellitus in a mammal with a beneficial effect on glycaemic control, which comprises 5-[4-[2-(N-methyl-N-(2-pyridyl) amino)ethoxy]benxyl]thiazolidine-2,4-dione (Compound I) or pharmaceutically acceptable form thereof in an amount of 2 to 12 mg, an insulin secretagogue and a biguanide antihyperglycaemic agent.

2. A composition according to claim 1, wherein the insulin secretagogue is glibenclamide, glipizide, gliclazide, glimepiride, tolazamide, tolbutamide, acetohexamide, carbutamide, chlorpropamide, glibornuride, gliquidone, glisentide, glisolamide, glisoxepide, glyclopyamide, glycylamide or repaglinide.

3. A composition according to claims 1, wherein the biguanide antihyperglycaemic agent is metformin, buformin or phenformin.

4. A pharmaceutical composition for the treatment of diabetes mellitus and conditions associated with diabetes mellitus in a mammal with a beneficial effect on glycaemic control, which comprises 2 to 12 mg 5-[4-[2-(N-methyl-N-(2-pyridyl)amino)ethoxy]benxyl]thiazolidine-2,4-dione (Compound I) or pharmaceutically acceptable form thereof, glimepiride and one of metformin, buformin or phenformin.

5. A composition according to any one of claims 1 to 4, which comprises 2 to 4, 4 to 8 or 8 to 12 mg of Compound (I).

6. A composition according to any one of claims 1 to 4, which comprises 2 to 4 mg of Compound (I).

7. A composition according to any one of claims 1 to 4, which comprises of 4 to 8 mg of Compound (I).

8. A composition according to any one of claims 1 to 4, which comprises of 8 to 12 mg of Compound (I).

9. A composition according to any one of claims 1 to 4, which comprises 2 mg of Compound (I).

10. A composition according to any one of claims 1 to 4, which comprises 4 mg of Compound (I).

11. A composition according to any one of claims 1 to 4, which comprises the administration of 8 mg of Compound (I).

12. Use of 5-[4-[2-(N-methyl-N-(2-pyridyl)amino)ethoxy]benxyl]thiazolidine-2,4-dione (Compound I) or pharmaceutically acceptable form thereof in an amount of 2 to 12 mg , an insulin secretagogue and a biguanide antihyperglycaemic agent for the preparation of a medicament for the treatment of diabetes mellitus and conditions associated with diabetes mellitus in a mammal with a beneficial effect on glycaemic control.

13. Use according to claim 12, wherein the insulin secretagogue is glibenclamide, glipizide, gliclazide, glimepiride, tolazamide, tolbutamide, acetohexamide, carbutamide, chlorpropamide, glibomuride, gliquidone, glisentide, glisolamide, glisoxepide, glyclopyamide, glycylamide or repaglinide.

14. Use according to claim 12 or 13, wherein the alpha glucosidase inhibitor antihyperglycaemic agent is metformin, buformin or phenformin.

15. Use of 5-[4-[2-(N-methyl-N-(2-pyridyl)amino)ethoxy]benxyl]thiazolidine-2,4-dione (Compound I) in an amount 2 to 12 mg or pharmaceutically acceptable form thereof, glimepiride and one of metformin, buformin or phenformin for the preparation of a medicament for the treatment of diabetes mellitus and conditions associated with diabetes mellitus in a mammal with a beneficial effect on glycaemic control.

16. Use according to any one of claims 12 to 15, which comprises 2 to4, 4 to 8 or 8 to 12 mg of Compound (I).

17. Use according to any one of claims 12 to 15, which comprises 2 to 4 mg of Compound (I).

18. Use according to any one of claims 12 to 15, which comprises 4 to 8 mg of Compound (I).

19. Use according to any one of claims 12 to 15, which comprises 8 to 12 mg of Compound (I).

20. Use according to any one of claims 12 to 15, which comprises 2 mg of Compound (I).

21. Use according to any one of claims 12 to 15, which comprises 4 mg of Compound (I).

22. Use according to any one of claims 12 to 15, which comprises the administration of 8 mg of Compound (I).

## Patentansprüche

1. Arzneimittel zur Behandlung von Diabetes mellitus und mit Diabetes mellitus verbundenen Zuständen bei einem Säuger mit einer vorteilhaften Wirkung auf die Steuerung des Blutzuckergehalts, welches 5-[4-[2-(N-Methyl-N-(2-pyridyl)amino)ethoxy]benxyl]thiazolidin-2,4-dion (Verbindung I) oder eine pharmazeutisch verträgliche Form davon in einer Menge von 2 bis 12 mg, ein Insulin-Sekretagogum und ein Biguanid-Mittel gegen Hyperglykämie umfasst.

2. Zusammensetzung nach Anspruch 1, wobei das Insulin-Sekretagogum Glibenclamid, Glipizid, Gliclazid, Glimepirid, Tolazamid, Tolbutamid, Acetohexamid, Carbutamid, Chlorpropamid, Glibomurid, Gliquidon, Glisentid, Glisolamid, Glisoxepid, Glyclopyamid, Glycylamid oder Repaglinid ist.

3. Zusammensetzung nach Anspruch 1, wobei das Biguanid-Mittel gegen Hyperglykämie Metformin, Buformin oder Phenformin ist.

4. Arzneimittel zur Behandlung von Diabetes mellitus und mit Diabetes mellitus verbundenen Zuständen bei einem Säuger mit einer vorteilhaften Wirkung auf die Steuerung des Blutzuckergehalts, welches 2 bis 12 mg 5-[4-[2-(N-Methyl-N-(2-pyridyl)amino)ethoxy]benxyl]thiazolidin-2,4-dion (Verbindung I) oder eine pharmazeutisch verträgliche Form davon, Glimepirid und eines von Metformin, Buförmin oder Phenformin umfasst.

5. Zusammensetzung nach einem der Ansprüche 1 bis 4, welche 2 bis 4, 4 bis 3 oder 8 bis 12 mg der Verbändung (1) umfasst.

6. Zusammensetzung nach einem der Ansprüche 1 bis 4, welche 2 bis 4 mg der Verbindung (I) umfasst.

7. Zusammensetzung nach einem der Ansprüche 1 bis 4, welche 4 bis 8 mg der Verbindung (I) umfasst.

8. Zusammensetzung nach einem der Ansprüche 1 bis 4, welche 8 bis 12 mg der Verbindung (I) umfasst.

9. Zusammensetzung nach einem der Ansprüche 1 bis 4, welche 2 mg der Verbindung (I) umfasst.

10. Zusammensetzung nach einem der Ansprüche 1 bis 4, welche 4 mg der Verbindung (I) umfasst.

11. Zusammensetzung nach einem der Ansprüche 1 bis 4, welche die Verabreichung von 8 mg der Verbindung (I) umfasst.

12. Verwendung von 5-[4-[2-(N-Methyl-N-(2-pyridyl)amino)ethoxy]benxyl]thiazolidin-2,4-dion (Verbindung I) oder eimer pharmazeutisch verträglichen Form davon in einer Menge von 2 bis 12 mg, eines Insulin-Sekretagogums und eines Biguanid-Mittels gegen Hyperglykämie zur Herstellung eines Medikaments zur Behandlung von Diabetes mellitus und mit Diabetes mellitus verbundenen Zuständen bei einem Säuger mit einer vorteilhaften Wirkung auf die Steuerung des Blutzuckergehalts.

13. Verwendung nach Anspruch 12, wobei das Insulin-Sekretagogum Glibenclamid, Glipizid, Gliclazid, Glimepirid, Tolazamid, Tolbutamid, Acetohexamid, Carbutamid, Chlorpropamid, Glibomurid, Gliquidon, Glisentid, Glisolamid, Glisoxepid, Glyclopyamid, Glycylamid oder Repaglinid ist.

14. Verwendung nach Anspruch 12 oder 13, wobei das α-Glucosidaseinhibitor-Mittel gegen Hyperglykämie Metformin, Buformin oder Phenformin ist.

15. Verwendung von 5-[4-[2-(N-Methyl-N-(2-pyridyl)amino)ethoxy]benxyl]thiazolidin-2,4-dion (Verbindung I) in einer Menge von 2 bis 12 mg oder einer pharmazeutisch verträglichen Form davon, von Glimepirid und einem von Metformin, Buformin oder Phenformin zur Herstellung eines Medikaments zur Behandlung von Diabetes mellitus und mit Diabetes mellitus verbundenen Zuständen bei einem Säuger mit einer vorteilhaften Wirkung auf die Steuerung des Blutzuckergehalts.

16. Verwendung nach einem der Ansprüche 12 bis 15, welche 2 bis 4, 4 bis 8 oder 8 bis 12 mg der Verbindung (I) umfasst.

17. Verwendung nach einem der Ansprüche 12 bis 15, welche 2 bis 4 mg der Verbindung (I) umfasst.

18. Verwendung nach einem der Ansprüche 12 bis 15, welche 4 bis 8 mg der Verbindung (I) umfasst.

19. Verwendung nach einem der Ansprüche 12 bis 15, welche 8 bis 12 mg der Verbindung (1) umfasst.

20. Verwendung nach einem der Ansprüche 12 bis 15, welche 2 mg der Verbindung (I) umfasst.

21. Verwendung nach einem der Ansprüche 12 bis 15, welche 4 mg der Verbindung (I) umfasst.

22. Verwendung nach einem der Ansprüche 12 bis 15, welche die Verabreichung von 8 mg der Verbindung (I) umfasst.

## Revendications

1. Composition pharmaceutique pour le traitement du diabète sucré et d'affections associées au diabète sucré chez un mammifère, avec un effet bénéfique sur le contrôle de la glycémie, qui comprend de la 5-[4-[2-(N-méthyl-N-(2-pyridyl)amino)éthoxy]benzyl]thiazolidine-2,4-dione (Composé I) ou une de ses formes pharmaceutiquement acceptables en une quantité de 2 à 12 mg, un sécrétagogue d'insuline et un agent antihyperglycémique du type biguanide.

2. Composition suivant la revendication 1, dans laquelle le sécrétagogue d'insuline est le glibenclamide, le glipizide, le gliclazide, le glimépiride, le tolazamide, le tolbutamide, l'acétohexamide, le carbutamide, le chlorpropamide, le glibornuride, le gliquidone, le glisentide, le glisolamide, le glisoxépide, le glyclopyamide, le glycylamide ou le répaglinide.

3. Composition suivant la revendication 1, dans laquelle l'agent antihyperglycémique du type biguanide est la metformine, la buformine ou la phenformine.

4. Composition pharmaceutique pour le traitement du diabète sucré et d'affections associées au diabète sucré chez un mammifère, avec un effet bénéfique sur le contrôle de la glycémie, qui comprend 2 à 12 mg de 5-[4-[2-(N-méthyl-N-(2-pyridyl)amino)éthoxy]benzyl]thiazolidine-2,4-dione (composé I) ou une de ses formes pharmaceutiquement acceptables, du glimépiride et un des agents consistant en metformine, buformine et phenformine.

5. Composition suivant l'une quelconque des revendications 1 à 4, qui comprend 2 à 4, 4 à 8 ou 8 à 12 mg du Composé (I).

6. Composition suivant l'une quelconque des revendications 1 à 4, qui comprend 2 à 4 mg du composé (I).

7. Composition suivant l'une quelconque des revendications 1 à 4, qui comprend 4 à 8 mg du Composé (I).

8. Composition suivant l'une quelconque des revendications 1 à 4, qui comprend 8 à 12 mg du Composé (I).

9. Composition suivant l'une quelconque des revendications 1 à 4, qui comprend 2 mg du Composé (I).

10. Composition suivant l'une quelconque des revendications 1 à 4, qui comprend 4 mg du Composé (I).

11. Composition suivant l'une quelconque des revendications 1 à 4, qui comprend l'administration de 8 mg du Composé (I).

12. Utilisation de 5-[4-[2-(N-méthyl-N-(2-pyridyl)-amino)éthoxy]benzyl]thiazolidine-2,4-dione (Composé I) ou d'une de ses formes pharmaceutiquement acceptables en une quantité de 2 à 12 mg, d'un sécrétagogue d'insuline et d'un agent antihyperglycémique du type biguanide pour la préparation d'un médicament destiné au traitement du diabète sucré et d'affections associées au diabète sucré chez un mammifère, avec un effet bénéfique sur le contrôle de glycémie.

13. Utilisation suivant la revendication 12, dans laquelle le sécrétagogue d'insuline est le glibenclamide, le glipizide, le gliclazide, le glimépiride, le tolazamide, le tolbutamide, l'acétohexamide, le carbutamide, le chlorpropamide, le glibornuride, la gliquidone, la glisentide, le glisolamide, le glisoxépide, le glyclopyamide, le glycylamide ou le répaglinide.

14. Utilisation suivant la revendication 12 ou 13, dans laquelle l'agent antihyperglycémique inhibiteur d'alpha-glucosidase est la metformine, la buformine ou phenformine.

15. Utilisation de 5-[4-[2-(N-méthyl-N-(2-pyridyl)-amino)éthoxy]benzyl]thiazolidine-2,4-dione (Composé I) en une quantité de 2 à 12 mg ou d'une de ses formes pharmaceutiquement acceptables, de glimépiride et d'un des agents consistant en metformine, buformine et phenformine pour la préparation d'un médicament destiné au traitement du diabète sucré et d'affections associées au diabète sucré chez un mammifère, avec un effet bénéfique sur le contrôle de la glycémie.

16. Utilisation suivant l'une quelconque des revendications 12 à 15, qui comprend 2 à 4, 4 à 8 ou 8 à 12 mg du Composé (I).

17. Utilisation suivant l'une quelconque des revendications 12 à 15, qui comprend 2 à 4 mg du composé (I).

18. Utilisation suivant l'une quelconque des revendications 12 à 15, qui comprend 4 à 8 mg du Composé (I).

19. Utilisation suivant l'une quelconque des revendications 12 à 15, qui comprend 8 à 12 mg du Composé (I).

20. Utilisation suivant l'une quelconque des revendications 12 à 15, qui comprend 2 mg du Composé (I).

21. Utilisation suivant l'une quelconque des revendications 12 à 15, qui comprend 4 mg du Composé (I).

22. Utilisation suivant l'une quelconque des revendications 12 à 15, qui comprend l'administration de 8 mg du Composé (I).
